# DEMANDE DE BREVET EUROPEEN

(11) **EP 1 389 476 A1**
(43) Date de publication de la demande: **18.02.2004**
(21) Numéro de dépôt: 02405693.9
(22) Date de dépôt: 14.08.2002
(51) Int. Cl.: A61M 5/142, F04B 43/12

(54) **Dispositif de programmation d'une pompe pour l'injection de medicaments**

(71) Demandeur: Precimedix S.A., 2000 Neuchâtel (CH)
(72) Inventeur: Möri, Peter, 3272 Walperswil (CH); Wüthrich, Heinz, 3048 Worblaufen (CH); Stulz, Beat, 1700 Fribourg (CH); Clement, Claude, 1333 Lussy-sur-Morges (CH)
(74) Mandataire: Gresset, Jean

(57) **Abrégé**

L'invention concerne un dispositif de programmation d'une pompe pour l'injection de médicaments, formée de deux modules accouplables, soit :
- un module cassette (12) contenant un liquide à injecter, qui comporte une mémoire électronique (36) destinée à contenir des données relatives au traitement que doit recevoir le patient, et
- un module pompe (10) qui comporte des moyens d'actionnement (18) agissant sur le module cassette (12) pour faire passer le liquide vers l'extérieur et un microprocesseur pour la commande desdits moyens à partir des données contenues dans ladite mémoire (36).

Ce dispositif comporte :
- un ordinateur (46) servant à produire lesdites données, et
- une première interface (50) pouvant être connectée à l'ordinateur pour recevoir ces données et conformée pour être accouplée au module cassette (12) à la place du module pompe (10) afin d'introduire les données dans sa mémoire (36).

## Description

La présente invention se rapporte au domaine des pompes miniaturisées pour l'injection de médicaments dans le corps d'un patient selon un programme prescrit. Elle concerne, plus particulièrement, un dispositif permettant de programmer une pompe médicale formée d'un module pompe et d'un module cassette contenant le médicament, qui s'accouplent de manière amovible.

Les pompes miniaturisées à usage médical sont connues depuis plusieurs années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit, pour autant, alité et relié à un appareil encombrant, coûteux et bruyant.

De telles pompes sont, souvent, de type péristaltique rotatif. Leur principe consiste à disposer d'un tuyau en matière plastique déformable relié à un réservoir contenant la solution de médicament et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et poussé vers la sortie pour être injecté dans le corps du patient.

Le document FR 2 753 235, par exemple, décrit des pompes à cassette de ce type.

Lors de la conception de ces pompes, il est particulièrement important de se préoccuper de leur facilité de programmation. Destinées surtout à être utilisées en milieu non hospitalier, elles doivent, en effet, pouvoir être mises en service, de manière sûre, par tout personnel soignant, voire par le patient lui-même, sans qu'il ait reçu une formation spéciale ou doive lire un long et compliqué mode d'emploi. Or, ces exigences de convivialité et de sécurité sont rarement satisfaites par les pompes actuellement sur le marché. Le risque est, alors, qu'elles restent un équipement d'hôpital réservé à quelques infirmières, ce qui leur fait perdre une grande partie de leur intérêt.

Le document EP-02 405639.2 décrit une pompe programmable à cassette immédiatement utilisable par toute personne, sans nécessiter aucune formation ou connaissance particulière. Elle est formée de deux modules accouplables, soit :
- un module cassette contenant un liquide à injecter, qui comporte une mémoire électronique destinée à contenir des données relatives au traitement que doit recevoir le patient, et
- un module pompe qui comporte des moyens d'actionnement agissant sur le module cassette pour faire passer le liquide vers l'extérieur, un microprocesseur pour la commande desdits moyens à partir des données contenues dans ladite mémoire et une source d'énergie électrique.

Pour qu'une pompe du type décrit ci-dessus réponde de manière optimale aux exigences de facilité d'utilisation et de sécurité, il est encore nécessaire que sa programmation elle-même soit réalisée au moyen d'un dispositif satisfaisant les mêmes exigences.

La présente invention a donc pour but de fournir un tel dispositif de programmation qui comporte :
- un ordinateur servant à produire les données relatives au traitement que doit recevoir le patient, et
- une interface pouvant être connectée à l'ordinateur pour recevoir ces données et conformée pour être accouplée au module cassette à la place du module pompe afin d'introduire les données dans sa mémoire.

De façon particulièrement avantageuse, le dispositif selon l'invention sert aussi à la programmation d'une pompe dont le module pompe comprend une mémoire électronique destinée à contenir des données de sécurité. Dans ce cas, le dispositif comporte une deuxième interface pouvant être connectée à l'ordinateur pour recevoir ces données de sécurité et conformée pour être accouplée au module pompe à la place du module cassette afin d'introduire les données dans sa mémoire. De préférence, lorsque la source d'énergie est un accumulateur, la deuxième interface sert aussi à sa recharge.

Dans un mode de réalisation préféré, le dispositif selon l'invention permet de programmer une pompe dont les modules pompe et cassette sont liés au moyen d'une articulation à baïonnette comportant une partie mâle et une partie femelle. Dans ce cas, la première interface comporte la même partie d'articulation que le module pompe, alors que la deuxième interface comporte la même partie d'articulation que le module cassette.

Le dispositif selon l'invention peut être utilisé, tout spécialement, pour la programmation d'une pompe dans laquelle la mémoire du module cassette est une carte de type SIM (Subscriber Information Memory) et le module pompe comporte un connecteur relié à son microprocesseur et positionné de manière à ce que, lors de l'accouplement des deux modules, ses plages de contact viennent précisément au contact des plages de ladite carte SIM. La première interface du dispositif comporte alors un connecteur positionné de manière à ce que, lorsque l'interface et le module cassette sont assemblés, ses plages de contact viennent précisément au contact de la carte SIM. Dans ce cas, la deuxième interface comporte un connecteur positionné de manière à ce que, lorsque l'interface et le module pompe sont assemblés, ses plages de contact viennent précisément au contact du connecteur du module pompe.

De façon très avantageuse, le dispositif selon l'invention comporte des moyens pour établir, à distance, un transfert de données bidirectionnel entre l'ordinateur et la mémoire du module cassette.

L'invention sera mieux comprise à la lecture de la description qui suit, faite en référence du dessin annexé, dans lequel :
- la figure 1 représente les deux interfaces du dispositif de programmation et la façon dont elles se connectent aux deux modules de la pompe programmable ;
- la figure 2 montre, très schématiquement, la configuration utilisée pour programmer les deux modules ; et
- la figure 3 illustre la manière de programmer la pompe par téléphone.

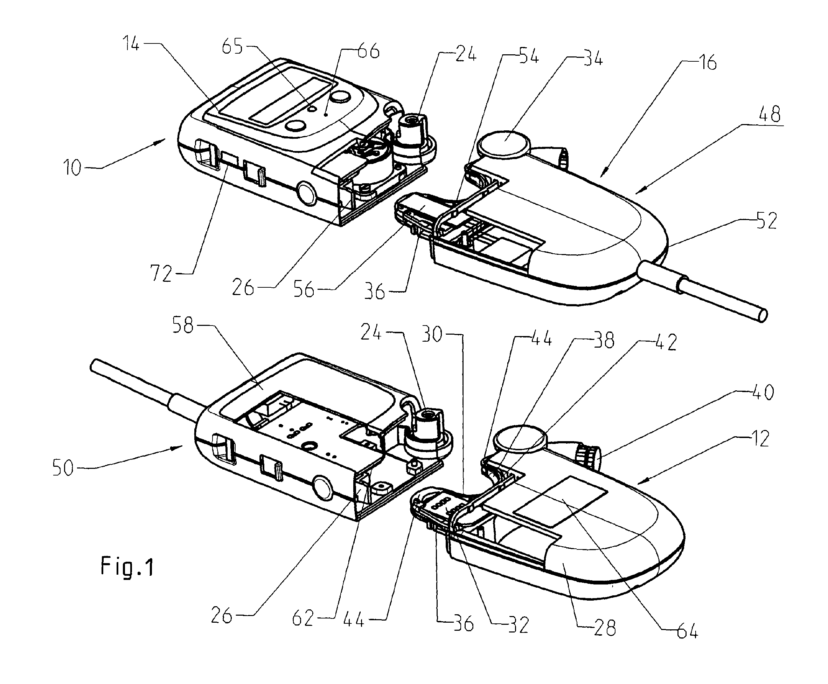

La figure 1 montre une pompe pour l'administration de médicaments, formée d'un module pompe 10 et d'un module cassette 12 qui s'accouplent l'un à l'autre de manière amovible au moyen d'une articulation à baïonnette. Sur la figure, les deux modules sont représentés séparément.

La pompe est celle qui fait l'objet de la demande de brevet EP susmentionnée. Elle ne sera donc pas décrite en détail.

On se contentera d'indiquer que le module pompe 10 laisse apparaître, du côté ouvert de son boîtier 14 :
- une platine métallique 16 qui sert de base à une tête de pompe 18 péristaltique rotative à trois galets presseurs ;
- l'extrémité d'un circuit imprimé 20 doté d'un connecteur 22 pour assurer la liaison électrique du module pompe avec l'extérieur et servant de support d'interconnexion à un microprocesseur et une mémoire destinée à enregistrer des données de sécurité ;
- la partie mâle 24 de l'articulation à baïonnette ; et
- la première partie 26 d'un verrou solidarisant les deux modules.

Le module pompe 10 abrite également un accumulateur (non visible sur la figure) et destiné à l'alimenter en énergie.

Le module cassette 12 comporte, à l'intérieur d'un boîtier 28, une contre-pièce 30 munie, du côté ouvert du boîtier :
- d'une carte mémoire 32, de type SIM (Subscriber Information Memory), destinée à enregistrer des données relatives au programme d'injection prescrit et l'historique de l'utilisation de la pompe ;
- de la partie femelle 34 de l'articulation à baïonnette ; et
- de la deuxième partie 36 du verrou.

Le module cassette 12, lui aussi décrit en détail dans le document EP susmentionné, renferme une poche réservoir de médicament (non visible sur la figure) qu'un tuyau souple 38 relie à un raccord 40 destiné à connecter une tubulure souple se terminant par une aiguille d'injection pour administrer au patient le médicament contenu dans la cassette.

Le tuyau souple 38 est appliqué sur la contre-pièce 30 qui occupe toute la largeur du boîtier 28 et est positionnée par une rainure de sa coque inférieure. La pièce 30 comporte, en son milieu, une partie concave arrondie 42, en forme de U, qui définit une zone d'appui sur laquelle, une fois les deux modules assemblés, lors de la rotation de la tête de pompe 18, les trois galets presseurs de celle-ci viennent tour à tour écraser le tuyau souple 38 et pousser ainsi vers l'extérieur, par un mouvement péristaltique, le liquide contenu dans la poche réservoir.

La partie concave 42 de la contre-pièce 30 est au milieu de deux parties convexes 44 dont l'une sert de support à la carte SIM 32 positionnée de manière à ce que, lorsque les deux modules de la pompe sont assemblés, ses plages de contact viennent précisément au contact des plages du connecteur 22 appartenant au module pompe 10.

La contre-pièce 30 possède, sous la carte SIM 32, la deuxième partie 36 du verrou et, à son extrémité opposée à celle qui porte la carte SIM, la partie femelle 34 de l'articulation à baïonnette.

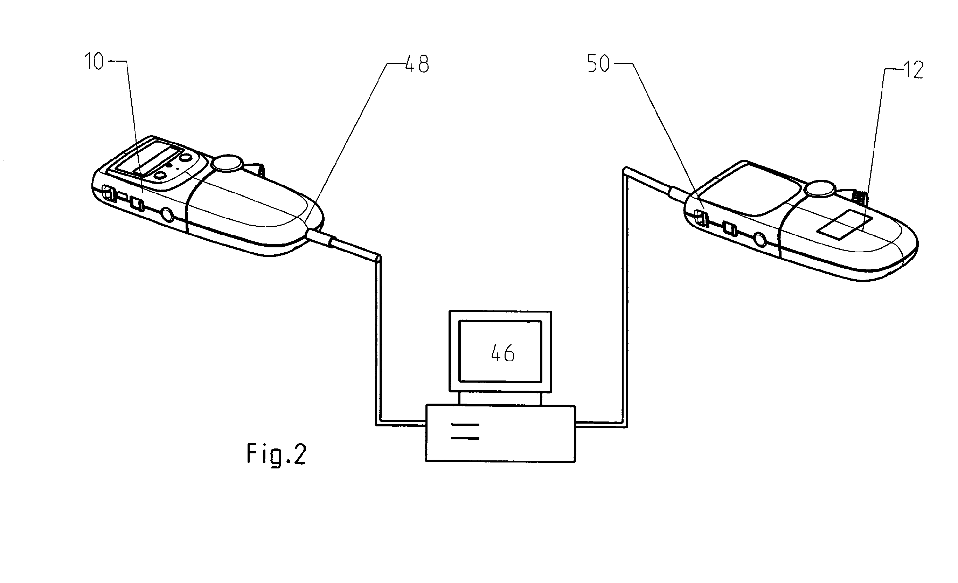

Selon l'invention, comme le montrent les figures 1 et 2, le dispositif de programmation comprend :
- un ordinateur 46 doté d'un logiciel lui permettant de communiquer avec les autres éléments du dispositif ;
- une première interface 48, dite interface pompe, destinée à relier l'ordinateur 46 et le module pompe 10 ; et
- une deuxième interface 50, dite interface cassette, destinée à relier l'ordinateur 46 et le module cassette 12.

L'interface pompe 48 comporte un boîtier 52 qui, pour des raisons économiques évidentes, est le même que le boîtier 28 du module cassette 12, mais sans nécessairement le raccord 40. Une pièce de liaison 54, identique à la contre-pièce 30 et positionnée par une rainure du boîtier 52, porte donc la deuxième partie 36 du verrou et la partie femelle 34 de l'articulation à baïonnette.

La pièce de liaison 54 sert de support, à la place de la carte SIM 32, à un connecteur 56 dont les plages de contact sont positionnées de manière à ce que, lorsque l'interface 48 et le module pompe 10 sont accouplés, elles viennent précisément au contact du connecteur 22 du module pompe.

L'ordinateur 46 est relié au connecteur 56, grâce auquel le circuit imprimé 20 du module pompe 10 peut être ainsi mis en communication avec l'ordinateur pour effectuer sa programmation et la recharge de son accumulateur.

L'interface cassette 50 comporte un boîtier 58 identique au boîtier 14 du module pompe 10. Il porte donc la première partie 26 du verrou et la partie mâle 24 de l'articulation à baïonnette.

Le boîtier 58 abrite un circuit imprimé 60 dont l'extrémité, placée au même endroit que celle du circuit imprimé 20 du module pompe, est dotée d'un connecteur 62 disposé de manière à ce que, lorsque l'interface 50 et le module cassette 12 sont assemblés, ses plages de contact viennent précisément se superposer à la carte SIM 32 portée par la contre-pièce 30 du module cassette.

L'ordinateur 46 est relié au connecteur 62, grâce auquel la carte SIM 32 du module cassette 12 peut être ainsi mise en communication avec l'ordinateur pour effectuer sa programmation.

L'ordinateur 46 a été programmé pour produire toutes les informations de sécurité qui peuvent être souhaitées par le milieu médical. Il s'agit, typiquement et principalement, des données suivantes :
- identification du patient ;
- instructions relatives à l'administration de différents médicaments susceptibles d'intervenir dans le traitement : débit d'injection instantané maximum, quantité injectable maximum par jour, nombre de bolus autorisés par jour, volume maximum de chaque bolus, espace minimum entre deux bolus, ... ;
- indication d'éventuelles contre-indications ou intolérances particulières à certains médicaments.

L'ordinateur 46 a aussi été programmé pour produire toutes les informations spécifiques au traitement que doit recevoir le patient. Il s'agit, typiquement et principalement, des données suivantes :
- identification du patient ;
- identification du médicament à injecter ;
- instructions relatives à l'administration du médicament pour le traitement considéré : programme d'injection (quels débits pendant quelles durées), débit des bolus, leur nombre et leur espacement autorisés, .....

On se référera maintenant plus spécialement à la figure 2 qui illustre la disposition des interfaces 48 et 50 d'une part, et des modules 10 et 12 d'autre part. Ainsi, lorsque l'on veut programmer les modules en vue de leur utilisation, et particulièrement lorsqu'il s'agit de la première utilisation du module pompe, on dispose :
- d'un module pompe 10, et
- d'un module cassette 12 sorti du stock de pharmacie et identifié par une étiquette de type code à barres 64, collée sur le boîtier 28 et comportant, en code et en clair, le type de médicament et son dosage (ex. : morphine 2g/L) ainsi que son volume (ex.: 30 mL).

Pour programmer le module pompe 10, on lui accouple l'interface pompe 48 alors reliée à l'ordinateur 46, comme s'il s'agissait de lui accoupler le module cassette 12. La mémoire du module pompe se trouve ainsi connectée à l'ordinateur 46, à l'aide duquel le personnel soignant peut simplement lui transférer les informations de sécurité désirées, telles que susmentionnées.

Simultanément, l'accumulateur du module pompe 10 est rechargé automatiquement. Avantageusement, un témoin 65 peut être disposé sur le boîtier 14 afin de fournir une information sur l'état de charge de l'accumulateur.

Pour programmer le module cassette 12, on lui accouple l'interface cassette 50 alors reliée à l'ordinateur 46, comme s'il s'agissait de lui accoupler le module pompe 10. La carte SIM 32 du module cassette se trouve ainsi connectée à l'ordinateur 46, à l'aide duquel le personnel soignant peut simplement lui transférer les informations spéciales relatives au traitement.

Les deux modules 10 et 12 se trouvent ainsi programmés en toute simplicité, la convivialité du logiciel permettant d'éviter au maximum toute erreur. Avantageusement, un certain nombre de données peuvent être préenregistrées dans le logiciel pour faciliter encore la programmation.

Pour une description du fonctionnement de la pompe lorsque les deux modules sont accouplés, on se référera, de nouveau, au document EP déjà cité à plusieurs reprises.

Selon l'invention, il est particulièrement avantageux que le microprocesseur du module pompe 10 soit programmé pour transférer à la carte SIM 32 du module cassette 12 l'historique de l'utilisation de la pompe, c'est-à-dire tous les changements d'état détectés : utilisation des BOLUS, ouverture de la pompe, arrêt de la pompe, modification du débit.... La carte SIM enregistre toutes ces informations, souvent importantes pour la suite de la thérapie, et ainsi, à la fin d'un cycle d'injection, le médecin peut y avoir accès. Il lui suffit, pour lire le contenu de la carte SIM 32, d'accoupler le module cassette 12 à l'interface cassette 50 alors reliée à l'ordinateur 46.

Il existe une autre possibilité de programmer la carte SIM 32 du module cassette 12, qui peut, notamment, être particulièrement avantageuse lorsque le patient est à son domicile et, ne supportant pas le traitement, demande au médecin de modifier le programme d'injection. Afin d'éviter un nouveau déplacement fatiguant pour le malade, il est alors possible au personnel soignant d'intervenir à distance par téléphone.

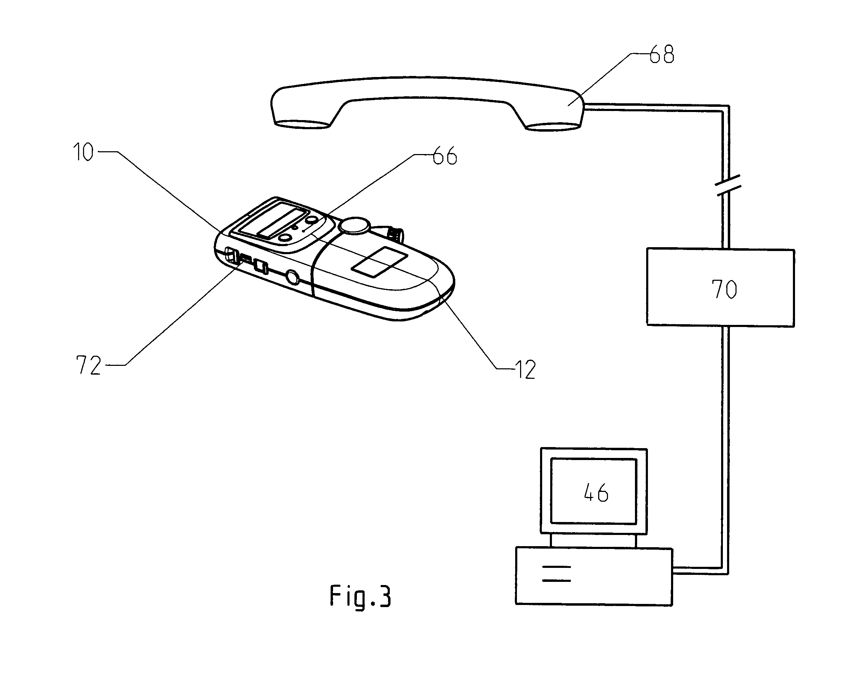

Pour permettre une telle intervention, ainsi qu'illustré schématiquement sur la figure 3, le module pompe 10 est équipé d'un microphone 66 relié à son microprocesseur et sur lequel le patient peut placer le haut-parleur de son téléphone 68. De son côté, le personnel soignant est équipé de l'ordinateur 46 connecté au réseau téléphonique par un modem 70.

Par une technique bien connue de l'homme de métier dans le domaine des télécommunications, l'ordinateur 46 peut ainsi transmettre au microphone 66 du module pompe 10 des informations codées correspondant à une modification du traitement. Les signaux ainsi produits par le microphone 66 sont reçus par le microprocesseur du module pompe 10 qui les transforme en données significatives pour la pompe. Les deux modules étant assemblés, ces données sont transférées, par le connecteur 22, à la carte SIM 32 du module cassette 12 afin de remplacer les données initiales relatives au programme prescrit. Dès cet instant, le module pompe 10 se met à fonctionner selon les nouvelles instructions reçues.

Dans ce mode de programmation par téléphone, il est avantageux, pour des raisons de sécurité, de doter le module pompe 10 d'un haut-parleur 72 et de programmer son microprocesseur pour émettre en direction du microphone du téléphone du patient des signaux acoustiques qui, après conversion, permettront de faire apparaître, sur l'écran de l'ordinateur 46, la confirmation que les instructions de modification du traitement ont été correctement reçues.

Enfin, il est possible d'intégrer, dans le module pompe 10, un module de téléphone GSM (Global System for Mobile communication) qui envoie directement au microprocesseur les données permettant de modifier le contenu de la mémoire SIM 32. Par rapport à la technique acoustique mentionnée précédemment, le système GSM permet d'échanger des informations avec un débit beaucoup plus important, tout en leur appliquant un codage de sécurité, ce qui autorise même l'envoi bidirectionnel de données privées relatives au patient.

## Revendications

1. Dispositif de programmation d'une pompe pour l'injection de médicaments dans le corps d'un patient, formée de deux modules accouplables, soit :
- un module cassette (12) contenant un liquide à injecter, qui comporte une mémoire électronique (36) destinée à contenir des données relatives au traitement que doit recevoir le patient, et
- un module pompe (10) qui comporte des moyens d'actionnement (18) agissant sur le module cassette (12) pour faire passer le liquide vers l'extérieur, un microprocesseur pour la commande desdits moyens à partir des données contenues dans ladite mémoire (36) et une source d'énergie électrique,
**caractérisé en ce qu'**il comporte :
- un ordinateur (46) servant à produire lesdites données, et
- une première interface (50) pouvant être connectée à l'ordinateur pour recevoir ces données et conformée pour être accouplée au module cassette (12) à la place du module pompe (10) afin d'introduire les données dans sa mémoire (36).

2. Dispositif selon la revendication 1, pour la programmation d'une pompe dont le module pompe (10) comprend une mémoire électronique destinée à contenir des données de sécurité, **caractérisé en ce qu'**il comporte une deuxième interface (48) pouvant être connectée à l'ordinateur (46) pour recevoir ces données et conformée pour être accouplée au module pompe (10) à la place du module cassette (12) afin d'introduire les données dans sa mémoire.

3. Dispositif selon la revendication 2, pour la programmation d'une pompe dans laquelle la source d'énergie est un accumulateur, **caractérisé en ce que** la deuxième interface (48) est dotée de moyens pour recharger ledit accumulateur.

4. Dispositif selon l'une des revendications 1 à 3, pour la programmation d'une pompe dont les modules pompe (10) et cassette (12) sont liés au moyen d'une articulation à baïonnette comportant une partie mâle et une partie femelle, **caractérisé** en ce la première interface (50) comporte la même partie (24) d'articulation que le module pompe (10), alors que la deuxième interface (48) comporte la même partie (34) d'articulation que le module cassette (12).

5. Dispositif selon l'une des revendications 1 à 4, pour la programmation d'une pompe dans laquelle la mémoire du module cassette (12) est une carte SIM (32) et le module pompe (10) comporte un connecteur (22) relié à son microprocesseur et positionné de manière à ce que, lors de l'accouplement des deux modules, ses plages de contact viennent précisément au contact des plages de ladite carte SIM, **caractérisé en ce que** ladite première interface (50) comporte un connecteur (62) positionné de manière à ce que, lorsque l'interface et le module cassette (12) sont assemblés, ses plages de contact viennent précisément au contact de la carte SIM (32).

6. Dispositif selon la revendication 5, **caractérisé en ce que** ladite deuxième interface (48) comporte un connecteur (56) positionné de manière à ce que, lorsque l'interface et le module pompe (10) sont assemblés, ses plages de contact viennent précisément au contact du connecteur (22) du module pompe.

7. Dispositif selon l'une des revendications 1 à 6, **caractérisé en ce qu'**il comporte des moyens (68, 70) pour établir, à distance, un transfert de données entre l'ordinateur (46) et la mémoire (36) du module cassette (12).

8. Dispositif selon la revendication 7, **caractérisé en ce que** ledit transfert de données est bidirectionnel.
